# EUROPÄISCHE PATENTSCHRIFT

(11) **EP 1 523 996 B1**
(45) Veröffentlichungstag und Bekanntmachung des Hinweises auf die Patenterteilung: **05.05.2010**
(21) Anmeldenummer: 04024025.1
(22) Anmeldetag: 08.10.2004
(51) Int. Cl.: A61L 2/12, A61L 2/26, A01M 1/22

(54) **Vorrichtung zum Sterilisieren von Gegenständen mittels Mikrowellen**
Device for sterilizing objects by means of microwaves
DIspositif pour la stérilisation d'objets au moyen de micro-ondes

(30) Priorität: 14.10.2003 DE 20315802 U
(43) Veröffentlichungstag der Anmeldung: 20.04.2005
(73) Patentinhaber: Zapf, Manfred, 87561 Oberstdorf (DE)
(72) Erfinder: Schiller, Detlef, 90768 Fürth (DE); Zapf, Manfred, 87561 Oberstdorf (DE)
(74) Vertreter: Hutzelmann, Gerhard

(56) Entgegenhaltungen:
- EP-A- 0 024 304
- DE-A- 3 719 994
- GB-A- 2 292 084
- US-A- 3 699 976
- US-A1- 2003 049 162

## Beschreibung

Die Erfindung bezieht sich auf eine Vorrichtung zum Sterilisieren von Gegenständen, insbesondere von Matratzen, Bettdecken, Holzmöbeln, Kleidung oder dergleichen.

Es ist eine Vielzahl verschiedener Vorrichtungen zum Sterilisieren von derartigen Gegenständen bekannt, die jedoch alle den Nachteil aufweisen, nur mit allergrößtem technischen Aufwand betrieben werden zu können. Eine Anwendung eines derartigen Sterilisators vor allem im häuslichen Bereich ist schon allein wegen der aufzuwendenden hohen Kosten in den allermeisten Fällen nicht möglich.

DE-A-3 719 994 offenbart eine Vorrichtung zur Sterilisierung von Holzmöbeln, die einen zerlegbaren Sterilisationsraum aufweist, dem eine Mikrowellen quelle zugeornet ist.

Aufgabe der Erfindung ist es eine Vorrichtung zum Sterilisieren von Gegenständen vorzuschlagen, welche bei geringem Kostenaufwand leicht anwendbar ist.

Diese Aufgabe wird erfindungsgemäß mit einer Vorrichtung gemäß Anspruch 1 gelöst.t.

Dadurch ist der Kostenaufwand gering. Mit der Mikrowellenstrahlung lassen sich auf einfache Art und Weise Milben, Flöhe, Bakterien, Holzwürmer oder dergleichen abtöten.

Es hat sich auch als sehr vorteilhaft erwiesen, wenn die Mikrowellenquelle als Magnetron ausgebildet und mit einer Abschirmung versehen ist.

Hierdurch wird die Mikrowellenstrahlung in Richtung des Sterilisationsraumes geleitet.

Eine weitere sehr vorteilhafte Ausgestaltung der Erfindung liegt auch darin, daß eine Einrichtung vorgesehen ist, welche die Mikrowellen zu unterbrechen vermag.

Damit kann die Mikrowellenstrahlung gepulst dem Sterilisationsraumes zugeführt werden.

Dabei hat es sich als vorteilhaft erwiesen, wenn ein zumindest metallisierter Propeller zum periodischen Unterbrechen der Mikrowellenstrahlung vorgesehen ist.

Hiermit lässt sich auf einfache Weise die Mikrowellenstrahlung unterbrechen.

Erfindungsgemäß sind die Wände des Sterilisationsraumes derart ausgebildet , daß diese Mikrowellenstrahlung reflektieren.

Hierdurch wird verhindert, daß Mikrowellen den Sterilisationsraum verlassen. Zusätzlich wird erreicht, daß der gesamte Sterilisationsraum von den Mikrowellen erreicht wird.

Erfindungsgemäß können die Wände des Sterilisationsraumes aus einer wenigstens partiell metallisierten Kunststoffolie und/oder einer Metallfolie aufgebaut sein.

Dadurch kann der Sterilisationsraum flexibel ausgebildet werden, wodurch er z.B. bei Nichtgebrauch zusammengelegt oder zusammengerollt werden kann.

Erfindungsgemäß können die Wände des Sterilisationsraumes aus einem Mikrowellen reflektierenden Gitternetz ausgebildet sein.

Ein Gitternetz sorgt für eine ausreichende Reflexion und Abschirmung, wobei jedoch Material eingespart werden kann. Der Sterilisator lässt sich dadurch noch flexibler einsetzen.

Die Wände des Sterilisationsraumes sind im Wesentlichen aus einem beliebigen festen oder flexiblen Material aufgebaut, welches metallisiert oder mit einer Mikrowellenstrahlung reflektierenden zweiten Schicht versehen ist.

Hiermit lassen sich auch feste Sterilisationsräume z.B. für stationären Einsatz bauen.

Gemäß einer weiteren Fortbildung der Erfindung hat es sich auch als sehr vorteilhaft erwiesen, wenn zwischen Mikrowellenquelle und Sterilisationsraum ein Gitter zur besseren Verteilung der Mikrowellenstrahlung vorgesehen ist.

Ein derartiges Gitter sorgt je nach seiner Maschenweite und geometrischen Form dafür, daß die von der Mikrowellenquelle abgegebene Strahlung durch Interferenzen und Überlagerungen möglichst gleichmässig im Sterilisationsraum verteilt wird.

Es hat sich auch als sehr vorteilhaft erwiesen, wenn zwei oder mehr Mikrowellenquellen vorgesehen sind, die an einer Seite des Sterilisationsraumes und/oder an verschiedenen Seiten des Sterilisationsraumes angeordnet sein können.

Hierdurch kann nahezu beliebig die Leistung des Sterilisators eingestellt werden. Zudem besteht die Möglichkeit, z.B. durch Anordnung von Mikrowellenquellen an gegenüberliegenden Seiten des Sterilisationsraumes eine besonders gute Ausleuchtung sicherzustellen.

Ebenfalls sehr vorteilhaft ist es, wenn eine Einrichtung vorgesehen ist, welche es erlaubt eine oder mehrere Mikrowellenquellen insbesondere entlang den Seiten des Sterilisationsraum automatisch oder manuell zu bewegen.

Dadurch kann eine oder mehrere Mikrowellenquellen über den zu sterilisierenden Gegenstand hinwegbewegt werden, wodurch sich auch sehr große Gegenstände, wie z.B. Matratzen sterilisieren lassen.

Desweiteren hat es sich als sehr vorteilhaft erwiesen, wenn eine Tür, Klappe oder eine sonstige Möglichkeit zum Öffnen und Verschließen des Sterilisationsraumes vorgesehen ist.

Hiermit lassen sich auf einfache Art Gegenstände in den Sterilisationsraum einbringen und auch wieder entnehmen

Eine weitere äußerst vorteilhafte Fortbildung der Erfindung ist auch darin zu sehen, daß eine Sicherheitseinrichtung vorgesehen ist, welche ein versehentliches Austreten von Mikrowellenstrahlung insbesondere bei nicht geschlossenem Sterilisationsraum verhindert.

Damit werden unbeabsichtigte Personenschäden wirksam verhindert.

Im Folgenden wird die Erfindung anhand eines Ausführungsbeispiels verdeutlich.

Dabei zeigen
- Fig. 1: eine schematische Darstellung eines erfindungsgemäßen Sterilisators und
- Fig. 2: eine schematische Darstellung eines weiteren erfindungsgemäßen Sterilisators.

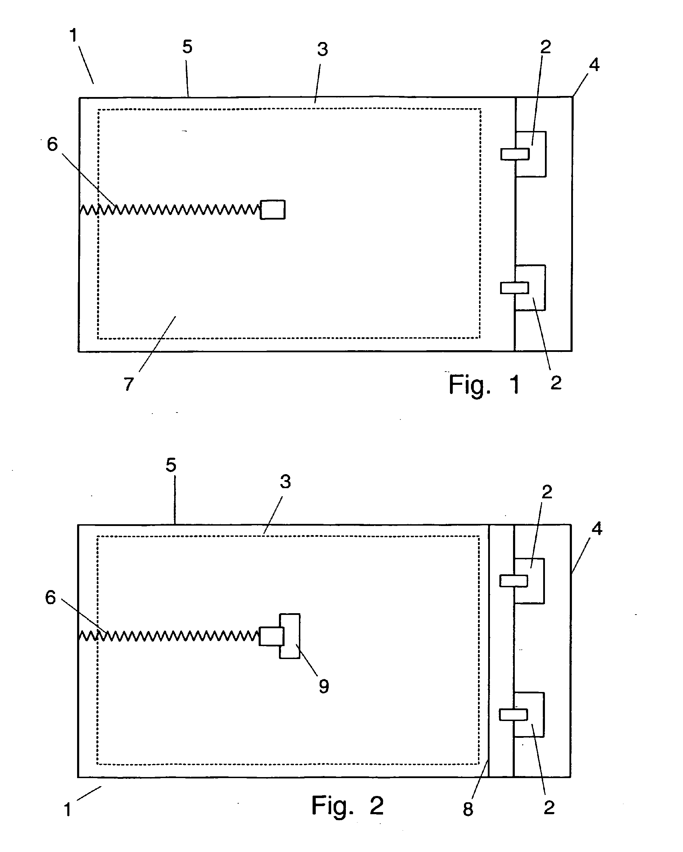

Mit 1 ist in Fig. 1 ein Sterilisator zur Abtötung von Milben, Flöhen, Läusen, Bakterien, Holzwürmern oder dergleichen mit einer Mikrowellenquelle 2 und einem Sterilisationsraum 3 bezeichnet.

Die Mikrowellenquelle 2 ist von einer Abschirmung 4 umgeben, welche dafür sorgt, daß die Mikrowellen nur in Richtung des Sterilisationsraumes 3 austreten können.

Der Sterilisationsraum 3 schließt direkt an die Abschirmung 4 der Mikrowellenquelle 2 an und ist zu dieser hin offen.

Der Sterilisationsraum 3 ist in alle anderen Richtungen von einer metallisierten Kunststoffolie 5 umgeben, welche die Mikrowellenstrahlung reflektiert und somit am Verlassen des Sterilisationsraumes 3 hindert.

Der Sterilisationsraum 3 lässt sich mittels eines Reißverschlusses 6 öffnen und kann dann beladen werden.

Der Sterilisator 1 eignet sich zum Sterilisieren von Matratzen, Bettdecken, Holzmöbeln, oder dergleichen.

Nachdem der Sterilisationsraum 3 mit Sterilisationsgut 7 beladen worden ist, wird der Reißverschluß 6 wieder verschlossen.

Zur Sterilisation wird die Mikrowellenquelle 2 für kurze Zeit aktiviert. Die Mikrowellenstrahlung sorgt dafür, daß die Schädlinge erhitzt und damit abgetötet werden.

Nach Abschalten der Mikrowellenquelle 2 kann das Sterilisationsgut 7 aus dem Sterilisationsraum 3 entnommen werden.

Als Mikrowellenquelle 2 kann ein handelsübliches Magnetron eingesetzt werden, wie es z.B. bei Mikrowellenherden Verwendung findet.

Es ist denkbar, daß vor der Austrittsöffnung der Mikrowellenquelle 2 ein Shutter d.h. eine Blende, welche die Austrittsöffnung der Mikrowellenquelle wenigstens zeitweise zu verschließen bzw. zu unterbrechen vermag, oder dergleichen angebracht ist.

Es ist auch denkbar, daß zwischen Mikrowellenquelle 2 und Sterilisationsraum 3 ein Gitter 8 angeordnet ist, welches durch Brechung, Interferenz und Überlagerung für eine gleichmäßige Ausleuchtung des Sterilisationsraumes 3 mit Mikrowellenstrahlung sorgt. Die Maschenweite und Geometrie des Gitters 8 wird auf die Wellenlänge der Mikrowellen und die Ausbildung des Sterilisationsraumes 3 angepasst. Das Gitter 8 kann aus einem Metall oder einem metallisiertem Material wie z.B. Kunststoff hergestellt sein.

Die Begrenzung des Sterilisationsraumes 3 kann auch aus einer Metallfolie, einem leitenden Gitter, oder auch festen Materialien wie z.B. Metall-, Kunststoff- oder Holzplatten gefertigt sein. Mikrowellen durchlässige Materialien müssen dabei mit einer Abschirmung aus einer Metallfolie, einer Metallisierung, einem Abschirmgitter oder dergleichen versehen sein.

Anstatt des Reißverschlusses 6 kann auch eine Türe oder dergleichen zum Öffnen des Sterilisationsraumes 3 vorgesehen sein.

Es ist auch denkbar, daß der Reißverschluß 6 oder aber auch eine Türe oder dergleichen eine Sicherheitseinrichtung 9 aufweist, welche die Mikrowellenquelle 2 abzuschalten vermag, wenn während des Betriebes des Sterilisators 1 der Sterilisationsraum 3 geöffnet wird.

Bei einer flexiblen Ausgestaltung des Sterilisationsraumes 3 ist es denkbar, daß der Sterilisator 1 bei Nichtgebrauch zusammengerollt wird und damit nur einen geringen Platzverbrauch bei der Lagerung aufweist.

Es können entlang des Sterilisationsraumes 3 auch mehrere Mikrowellenquellen 2 vorgesehen sein, welche insbesondere bei gegenüberliegender Anordnung eine besonders gute Ausleuchtung des Sterilisationsraumes 3 sicherstellen.

Desweiteren ist es auch denkbar, daß die Mikrowellenquelle 2 eventuell zusammen mit ihrer Abschirmung 4 über den Sterilisationsraum bzw das Sterilisationsgut 7 hinwegbewegt wird und damit das Sterilisationsgut 7 während der Einschaltdauer komplett überstreicht.

## Patentansprüche

1. Vorrichtung(1) zum Sterilisieren von Gegenständen(7)mit einem Sterilisationsraum(3) und einer Mikrowellenquelle(2), wobei die Wände des Sterilisationsraumes(3) derart ausgebildet sind, daß diese Mikrowellenstrahlung reflektieren, wobei die Wände des Sterilisationsraumes(3) aus einem beliebigen flexiblen Material aufgebaut sind, welches metallisiert oder mit einer Mikrowellenstrahlung reflektierenden zweiten Schicht versehen ist, wodurch der Sterilisationsraum zusammenrollbar ist, und wobei die Wände des Sterilisationsraumes(3) aus einer wenigstens partiell metallisierten Kunststoffolie(5), einer Metallfolie und/oder aus einem Mikrowellen reflektierenden Gitternetz aufgebaut sind.

2. Vorrichtung nach Anspruch 1, **dadurch gekennzeichnet**, das die Mikrowellenquelle(2) als Magnetron ausgebildet und mit einer Abschirmung(4) versehen ist.

3. Vorrichtung nach Anspruch 1 oder 2, **dadurch gekennzeichnet, daß** eine Einrichtung vorgesehen ist, welche die Mikrowellen zu unterbrechen vermag.

4. Vorrichtung nach Anspruch 3, **dadurch gekennzeichnet, daß** ein zumindest metallisierter Propeller zum periodischen Unterbrechen der Mikrowellenstrahlung vorgesehen ist.

5. Vorrichtung nach einem der vorangehenden Ansprüche, **dadurch gekennzeichnet, daß** zwischen Mikrowellenquelle(2) und Sterilisationsraum(3) ein Gitter(8) zur besseren Verseilung der Mikrowellenstrahlung vorgesehen ist

6. Vorrichtung nach einem der vorangehenden Ansprüche, **dadurch gekennzeichnet, daß** zwei oder mehr Mikrowellenquellen(2) vorgesehen sind, die an einer Seite des Sterilisationsraumes(3) und/oder an verschiedenen Seiten des Sterilisationsraumes(3) angeordnet sein können.

7. Vorrichtung nach einem der vorangehenden Ansprüche, **dadurch gekennzeichnet, daß** eine Einrichtung vorgesehen ist, welche es erlaubt eine oder mehrere Mikrowellenquellen(2) insbesondere entlang den Seiten des Sterilisationsraum(3) automatisch oder manuell zu bewegen.

8. Vorrichtung nach einem der vorangehenden Ansprüche, **dadurch gekennzeichnet, daß** eine Tür, Klappe oder eine sonstige Möglichkeit(6) zum Öffnen und Verschließen des Sterilisationsraumes(3) vorgesehen ist.

9. Vorrichtung nach einem der vorangehenden Ansprüche, **dadurch gekennzeichnet, daß** eine Sicherheitseinrichtung(9) vorgesehen ist, welche ein versehentliches Austreten von Mikrowellenstrahlung insbesondere bei nicht geschlossenem Sterilisationsraum(3) verhindert.

## Claims

1. Device (1) for sterilising articles (7), comprising a sterilisation chamber (3) and a microwave source (2), wherein the walls of the sterilisation chamber (3) are constructed in such a manner that these reflect microwave radiation, wherein the walls of the sterilisation chamber (3) are formed from a desired flexible material which is metallised or provided with a second layer reflecting microwave radiation, whereby the sterilisation chamber can be rolled up and wherein the walls of the sterilisation chamber (3) are formed from an at least partially metallised plastics material film (5), a metal foil and/or a netting reflecting microwaves.

2. Device according to claim 1, **characterised in that** the microwave source (2) is constructed as a magnetron and provided with a screen (4).

3. Device according to claim 1 or 2, **characterised in that** equipment capable of interrupting the microwaves is provided.

4. Device according to claim 3, **characterised in that** an at least one metallised propeller for periodic interruption of the microwave radiation is provided.

5. Device according to any one of the preceding claims, **characterised in that** a grid (8) for better distribution of the microwave radiation is provided between microwave source (2) and sterilisation chamber (3).

6. Device according to any one of the preceding claims, **characterised in that** two or more microwave sources (2) are provided, which can be arranged at one side of the sterilisation chamber (3) and/or at different sides of the sterilisation chamber (3).

7. Device according to any one of the preceding claims, **characterised in that** equipment allowing automatic or manual movement of one or more microwave sources (2), in particular along the sides of the sterilisation chamber (3) is provided.

8. Device according to any one of the preceding claims, **characterised in that** a door, flap or other possibility (6) for opening and closing the sterilisation chamber (3) is provided.

9. Device according to any one of the preceding claims, **characterised in that** safety equipment (9) preventing inadvertent issue of microwave radiation, particularly when the sterilisation chamber (3) is not closed, is provided.

## Revendications

1. Dispositif (1) de stérilisation d'objets (7) comportant une chambre de stérilisation (3) et une source de micro-ondes (2), les parois de la chambre de stérilisation (3) étant réalisées de façon à réfléchir le rayonnement micro-ondes, les parois de la chambre de stérilisation (3) étant formées d'un matériau flexible quelconque qui est métallisé ou muni d'une deuxième couche réfléchissant le rayonnement micro-ondes, de sorte que la chambre de stérilisation peut être enroulée, et les parois de la chambre de stérilisation (3) étant formées d'un feuille en matière plastique (5) au moins partiellement métallisée, d'une feuille métallique et/ou d'un réseau en grille réfléchissant les micro-ondes.

2. Dispositif selon la revendication 1, **caractérisé en ce que** la source de micro-ondes (2) est réalisée sous la forme d'un magnétron et pourvue d'un blindage ou écran (4).

3. Dispositif selon la revendication 1 ou 2, **caractérisé en ce qu'**il est prévu un équipement qui est capable d'interrompre les micro-ondes.

4. Dispositif selon la revendication 3, **caractérisé en ce qu'**il est prévu une hélice au moins métallisée pour interrompre périodiquement le rayonnement micro-ondes.

5. Dispositif selon une des revendications précédentes, **caractérisé en ce qu'**il est prévu une grille (8) entre la source de micro-ondes (2) et la chambre de stérilisation (3) pour une meilleure répartition du rayonnement micro-ondes.

6. Dispositif selon une des revendications précédentes, **caractérisé en ce qu'**il est prévu deux ou plus sources de micro-ondes (2) qui peuvent être disposées sur un côté de la chambre de stérilisation (3) et/ou sur des côtés différents de la chambre de stérilisation (3).

7. Dispositif selon une des revendications précédentes, **caractérisé en ce qu'**est prévu un équipement qui permet de déplacer automatiquement ou manuellement une ou plusieurs sources de micro-ondes (2) en particulier le long des côtés de la chambre de stérilisation (3).

8. Dispositif selon une des revendications précédentes, **caractérisé en ce qu'**il est prévu une porte, un clapet ou une autre possibilité (6) pour ouvrir et fermer la chambre de stérilisation (3).

9. Dispositif selon une des revendications précédentes, **caractérisé en ce qu'**il est prévu un équipement de sécurité (9) qui empêche une sortie accidentelle du rayonnement micro-ondes, en particulier lorsque la chambre de stérilisation (3) n'est pas fermée.
